Europäisches Patentamt

European Patent Office

Office européen des brevets

(11) Publication number: **0 393 969**
**A2**

(12) **EUROPEAN PATENT APPLICATION**

(21) Application number: 90304080.6

(22) Date of filing: 17.04.90

(51) Int. Cl.5: **C12N 15/53, C12N 15/62, C12N 15/75, C12P 19/40, //(C12N15/53,C12R1:07)**

The applicant has filed a statement in accordance with Rule 28 (4) EPC (issue of a sample only to an expert). Accession number(s) of the deposit(s): FERM BP-2378, FERM BP-2379, FERM BP-2380, FERM BP-2381 and FERM BP-2382.

(30) Priority: **19.04.89 JP 101084/89**

(43) Date of publication of application:
**24.10.90 Bulletin 90/43**

(84) Designated Contracting States:
**AT BE CH DE DK ES FR GB GR IT LI LU NL SE**

(71) Applicant: **TAKEDA CHEMICAL INDUSTRIES, LTD.**
**3-6, Doshomachi 2-chome Chuo-ku**
**Osaka 541(JP)**

(72) Inventor: **Miyagawa, Kenichiro**
**6-11, Higashitokiwadai 6-chome, Toyono-cho**
**Toyono-gun, Osaka 663-01(JP)**
Inventor: **Kanzaki, Naoyuki**
**17-201, 8 Kuragauchi 2-chome**
**Ibaraki, Osaka 567(JP)**
Inventor: **Hasegawa, Tatsuo**
**1-302, 4 Aomadani-nishi 1-chome**
**Minoo, Osaka 562(JP)**

(74) Representative: **Lewin, John Harvey et al**
**ELKINGTON AND FIFE Beacon House 113 Kingsway**
**London WC2B 6PP(GB)**

(54) DNA containing IMP dehydrogenase gene and its use.

(57) A DNA containing IMP dehydrogenase gene of the chromosome of a microorganism belonging to the genus Bacillus wherein a promoter of the IMP dehydrogenase gene is replaced with a different expressible promoter is disclosed. A vector wherein the DNA is integrated, a microorganism belonging to the genus Bacillus being capable of producing inosine and/or guanosine which is transformed with the DNA or the vector, and a process for producing inosine and/or guanosine which comprises cultivating the microorganism of the genus Bacillus in a culture medium to produce and accumulate inosine and/or guanosine and collecting the resultant are also disclosed.

# DNA CONTAINING IMP DEHYDROGENASE GENE AND ITS USE

## FIELD OF THE INVENTION

The present invention relates to an improved process for producing inosine and/or guanosine which are substances of importance as raw materials for the production of $5'$-inosinic acid ($5'$-IMP) which is a flavor component of "katsuobushi (dried bonito)" and $5'$-guanylic acid ($5'$-GMP) which is a flavor component of "shiitake mushroom", respectively, as well as a novel microorganism to be used for the process, and a novel DNA to be used for the derivation of the novel microorganism.

## BACKGROUND OF THE INVENTION

For the fermentative production of inosine, there have been known various processes using bacteria belonging to the genera Bacillus (U.S. Patent No. 3,960,661, Japanese Patent Publication No. 57-41915 and U.S. Patent 4,701,413), Brevibacterium (U.S. Patent No. 3,736,228) and the like having adenine-requiring character and, in addition, optionally provided with various drug resistance. Likewise, for the production of guanosine, there have been known various processes using bacteria belonging to the genera Bacillus (U.S. Patent No. 3,912,587 and U.S. Patent No. 4,283,346), Brevibacterium (Japanese Patent Publication No. 58-17592) and the like. However, since inosine and guanosine are produced through a common biosynthetic pathway, sometimes, guanosine is produced together with inosine during cultivation of a inosine-producer or, to the contrary, a large amount of inosine is produced together with guanosine by a guanosine-producer. And, in order to isolate inosine or guanosine from a culture broth, complicated purification processes are required.

Under these circumstances, there has been proposed a process for producing guanosine using a transformant containing a plasmid wherein a DNA coding for IMP dehydrogenase gene which plays the most important role in the guanosine biosynthetic pathway is ligated to the plasmid by utilizing recently developed recombinant DNA techniques (U.S. Patent No. 4,749,650).

On the other hand, there has been also proposed a process for deriving an IMP dehydrogenase defective strain by utilizing recombinant DNA techniques for inhibiting the formation of guanosine as a by-product (European Patent Publication No. 273660 A).

However, even the latter two processes are not always satisfactory compared with importance of inosine and guanosine in food industry. That is, the former process is not always satisfactory in view of stability of the production because it uses the plasmid, and the latter process requires complicated continuous feeding of xanthine which is a required substance for growth of the microorganisms. Therefore, a further improved process for producing inosine and/or guanosine has been desired.

## OBJECTS OF THE INVENTION

One object of the present invention is to provide an improved process for producing inosine and/or guanosine.

Another object of the present invention is to provide a novel microorganism belonging to the genus Bacillus to be used for the process of the present invention.

Still another object of the present invention is to provide a novel DNA containing IMP dehydrogenase gene to be used for the derivation of the novel microorganism of the present invention.

These objects as well as other objects and advantages of the present invention will be apparent to those skilled in the art from the following description with reference to the attached drawings.

## BRIEF EXPLANATION OF THE DRAWINGS

Fig. 1 illustrates the construction of the plasmid pEX214 in Example 1 as described hereinafter.

Figs. 2-1 to 2-3 illustrate the DNA sequence of IMP dehydrogenase gene.

Fig. 3 illustrates the construction of the plasmid pEX214Δ3 in Example 1 as described hereinafter and the DNA sequence in the neighborhood of the ligation site located in 5´-upstream of IMP dehydrogenase gene.

Fig. 4 illustrates the construction of the plasmid pEX241 in Example 1 as described hereinafter.

Fig. 5 illustrates the construction of the plasmid pEX242 in Example 1 as described hereinafter.

Fig. 6 illustrates the construction of the plasmid pEX210 in Example 2 as described hereinafter.

In the drawings, the symbols P, X, H, Sm, S, E, Sa, K, C, Ms, Mb, N and M represent cleavage sites by restriction enzymes PstI, XbaI, HincII, SmaI, SspI, EcoRI, SacI, KpnI, ClaI, MspI, MboI, NruI and MluI, respectively. The symbol (f) shows that its end is converted into a blunt end. The shaded part is IMP dehydrogenase gene region, the blank part is the adjacent region of IMP dehydrogenase gene, the black colored part is SPO1-26 promoter and the thick solid line is chloramphenicol-acetyltransferase gene region, and the solid line is the vector region.


# SUMMARY OF THE INVENTION


The present inventors have aimed at the fact that IMP dehydrogenase activity of a producer is greatly concerned with an amount of guanosine accumulated, and have studied intensively to increase a yield of guanosine or inosine by high expression of the gene of the enzyme to selectively accumulate a remarkable amount of guanosine or, to the contrary, by low expression of the gene to selectively accumulate a remarkable amount of inosine.

As a result, by using recombinant DNA techniques, the present inventors have succeeded in the derivation of a novel microorganism from a microbial strain being capable of producing inosine or both inosine and guanosine, wherein a promoter portion of a DNA coding for IMP dehydrogenase gene on the chromosome is replaced with a different promoter which permits high expression of the gene in the microbial strain and, on the other hand, have also succeeded in the derivation of another novel microorganism from a microbial strain being capable of producing inosine and guanosine, or inosine and xanthosine, or xanthosine and/or guanosine, wherein a promoter portion of a DNA coding for IMP dehydrogenase gene on the chromosome is replaced with a different promoter which permits low expression of the gene in the microbial strain.

Then, it has been found that a novel microorganism prepared by using a microorganism selected from bacteria belonging to the genus Bacillus as a recipient according to the above technique can accumulate a large amount of guanosine (or inosine) because IMP dehydrogenase gene having a promoter which permits the high (or low) expression is integrated on its chromosomal DNA and, therefore, a very stable production of inosine and/or guanosine can be carried out, repeatedly.

The present invention has been completed based on the above finding and is to provide a DNA containing IMP dehydrogenase gene of the chromosome of a microorganism belonging to the genus Bacillus wherein the promoter of the IMP dehydrogenase gene is replaced with a different expressible promoter. Further, the present invention is to provide a vector wherein the DNA of the present invention is integrated, a microorganism belonging to the genus Bacillus being capable of producing inosine and/or guanosine which is transformed with the DNA or the vector of the present invention, and a process for producing inosine and/or guanosine which comprises cultivating the microorganism of the genus Bacillus of the present invention in a culture medium to produce and accumulate inosine and/or guanosine and collecting the resultant.


# DETAILED DESCRIPTION OF THE INVENTION


A DNA containing an IMP dehydrogenase gene region used in the present invention is preferably that containing all the parts of DNA coding for the gene of the enzyme and, further, some peripheral parts forward and backward thereof. However, it may be a DNA containing a 5´-upstream part of the open reading frame containing a translation initiation site of the enzyme, SD sequence, a promoter and, further, some 5´-upstream region thereof.

Such a DNA is conveniently isolated from a chromosomal DNA of a microorganism by using recombinant DNA techniques. As a host-vector system for this purpose, EK system which is commonly employed can be suitably used. That is, as a host, there can be suitably used strains derived from Escherichia coli K-12 strain, for example, Escherichia coli C600 (T. Maniatis et al., Molecular Cloning, A Laboratory Manual, Cold Spring Harbor Laboratory, published by Cold Spring Harbor Laboratory Press, 1982, page 504), Escherichia coli JA221 (IFO 14359, ATCC 33875), Escherichia coli MM294 (ATCC 33625) [Proc. Natl. Acad. Sci. USA, 73, 4174 (1976)] or derivatives thereof. As a vector, there can be suitably used pBR322, pACYCl84, pACYCl77, pUC18, pUC19, pHSG396, pHSG298 and the like.

Further, other host-vector systems such as combinations of phage vectors such as Charon 4A (Molecular Cloning, page 31), EMBL 3 and EBML 4 (J. Mol. Biol., 170, 827) and the like to Escherichia coli DP 50 supF (Molecular Cloning, page 504), Escherichia coli NM 538 and NM 539 (J. Mol. Biol., 170, 827), Escherichia coli LE 392 (J. Biol. Chem., 218, 97) and the like can be used. Of course, a host-vector system wherein a microbe of the genus Bacillus, for example, Bacillus subtilis MI 114 (Gene, 24, 255) or its mutant is used as the host and a plasmid having capability of autonomous replication in a microorganism of the genus Bacillus such as pUB110, pC194, pE194, pS194, pSA2100, pHY300PLK or the like is used as the vector can be also used.

In principle, the origin of a donor microorganism of IMP dehydrogenase gene is not specifically limited in so far as the base sequence of IMP dehydrogenase gene of the microorganism has high homology to that of IMP dehydrogenase gene on the chromosome of a microorganism of the genus Bacillus to be used as a recipient as described hereinafter, and any microorganism can be used as the donor. Among them, when a microorganism of the genus Bacillus is used, so-called self cloning is realized and it is preferred because of easy handling. Further, the resulting transformant is expected to be stable. Furthermore, the donor is not necessarily capable of producing inosine, guanosine, xanthosine or purine bases thereof. However, preferably, the donor does not have IMP dehydrogenase deficiency.

Examples of such a DNA donor microorganism include Bacillus subtilis, Bacillus pumilus or Bacillus licheniformis. More particularly, the following microbial strains can be exemplified: Bacillus subtilis No. 168 (BGSCIAI), Bacillus subtilis No. 115 (IFO 14187, FERM BP-1327), Bacillus subtilis MI 114 (Gene, 24, 255), Bacillus pumilus ATCC 7061 and Bacillus licheniformis ATCC 14580 (wherein BGSC is The Bacillus Genetic Stock Center, IFO is Institute of Fermentation, Osaka and ATCC is The American Type Culture Collection).

The isolation of a DNA containing IMP dehydrogenase gene from these donors can be carried out according to the method described in U.S. Patent No. 4,749,650. Alternatively, according to the method described in European Patent Publication No. 273660 A, a DNA containing IMP dehydrogenase gene can be obtained by selecting a recombinant which complements xanthine requirement of a IMP dehydrogenase deficient mutant of a microorganism of the genus Bacillus, which has a chromosome of high homology to the DNA sequence of IMP dehydrogenase gene of a microorganism of the genus Bacillus to be used as a recipient as described hereinafter, from the gene library of the donor microorganism.

For the preparation and isolation of the novel DNA of the present invention from the DNA containing IMP dehydrogenase gene thus obtained, recombinant DNA techniques are conveniently employed and, for example, a host-vector system of Escherichia coli can be suitably used.

The novel DNA of the present invention can be prepared and isolated from a plasmid containing IMP dehydrogenase gene as follows.

In the following preparation steps of the DNA of the present invention in Escherichia coli, in many cases, it is convenient to subclone a DNA fragment in a new vector or to ligate it to downstream of a polylinker because of experimental convenience. For this purpose, it is preferable to carry out subcloning by utilizing, for example, a polylinker portion of pUC119 which is a vector of Escherichia coli because both the preparation of the novel DNA and the determination of the DNA sequence can be carried out, conveniently.

Firstly, a DNA fragment wherein a promoter region has been deleted from a DNA containing IMP dehydrogenase gene is prepared. This can be carried out, for example, as follows:

A plasmid containing IMP dehydrogenase gene is cleaved with a restriction enzyme having only one cleavage site located in upstream from the promoter and at a position close to the promoter. Then, the upstream region of IMP dehydrogenase gene is deleted by digesting the cleaved plasmid from the cleavage site with an enzyme which digests a double stranded DNA from its end in order, for example, BAL31 to obtain a shortened DNA fragment. In this case, for obtaining a DNA fragment wherein only just the promoter of IMP dehydrogenase gene is deleted, the desired DNA fragment can be selected by varying an amount of the enzyme used and a reaction time to obtain various DNA fragments having different lengths, and selecting the desired one by suitable means as described hereinafter, for example, the results of DNA sequence determination of the fragments. The determination of DNA sequence can be carried out by subcloning the DNA fragment in a vector for the determination of DNA sequence, for example, M13, pUC118 or pUC 119, and then using a known method, for example, the method of F. Sanger et al. (Proc.

Natl. Acad. Sci. USA, 74, 5463).

Then, the insertion of a different promoter into upstream from the open reading frame (upstream from SD sequence) of IMP dehydrogenase gene in the DNA thus obtained can be carried out, for example, as follows. The plasmid is cleaved with a restriction enzyme having only one cleavage site located in upstream from the open reading frame (upstream from SD sequence) of a plasmid containing IMP dehydrogenase gene wherein the promoter has been deleted according to a conventional method. On the other hand, a DNA fragment which shows a promoter activity in a recipient as described hereinafter is cut out from a DNA containing the fragment and, if necessary, it is separated with, for example, agarose gel electrophoresis and extracted and isolated. Then, the cleaved plasmid and the DNA fragment are mixed and, when the cohesive ends are the same, they are ligated with T4DNA ligase as they are. When the ends are different, they are converted into blunt ends with T4DNA polymerase and then ligated with T4DNA ligase. Thus, the desired DNA is obtained.

In this case, if a part of the 5′-adjacent region of IMP dehydrogenase gene is ligated to 5′-upstream of the replaced promoter, it is convenient because double crossing-over type recombination is caused upon recombination on chromosomes of a recipient as described hereinafter.

As the restriction enzyme for cleaving a plasmid containing IMP dehydrogenase gene, there can be used any restriction enzyme having a cleavage site in a upstream region of IMP dehydrogenase gene. Particularly, the enzyme which has a cleavage site located in 5′-upstream from the promoter of IMP dehydrogenase gene and at a position close to the promoter, and does not cleave any other part of the plasmid is preferably selected. Further, in the case of insertion of a different promoter, the enzyme which has a cleavage site located in a 5′-part of the open reading frame and SD sequence of IMP dehydrogenase gene and at a position close thereto, and does not cleave any other part of the plasmid is preferably selected as the restriction enzyme for cleaving the plasmid containing IMP dehydrogenase wherein the promoter has been deleted. For example, there can be suitably used AflII, ApaI, AxyI, BamHI, BclI, BglII, BstEII, ClaI, EcoRI, Eco81I, HpaI, HindIII, KpnI, MluI, NcoI, PstI, SacI, SacII, SalI, SmaI, SphI, SpII, SspI, StuI, XbaI, XhoI and the like.

The promoter used in the present invention is not limited to a specific one in so far as it has a DNA sequence which can be expressed in a recipient to be used in the later step, regardless of its origin, i.e., prokaryote, eukaryote or synthetic DNA. Examples thereof include promoters derived from chromosomes of bacteria belonging to the genus Bacillus, phage of bacteria belonging to the genus Bacillus, or plasmids having capability of autonomous growth in cells of bacteria belonging to the genus Bacillus, which permits stronger expression than that of IMP dehydrogenase gene of the chromosome of a microorganism belonging of the genus Bacillus (high expression), or permits weaker expression than that of IMP dehydrogenase gene of the chromosome of a microorganism belonging to the genus Bacillus (low expression). More particularly, examples of the promoter for high expression include those having at least, 7.5 nmol of XMP formed/min. mg.protein in the case of normal IMP dehydrogenase activity of 1.5 nmol of XMP formed/min. mg protein as measured by the method described by Magasanik (Magasanik, B., pages 106-111 of "Method in Enzymology", Vol. VI, published by Academic Press, New York, U.S.A.). Examples of the promoter for low expression include those having IMP dehydrogenase activity of, at most, 0.5 nmol of XMP formed/min. mg protein as measured under the same conditions. Specific examples thereof are as follows:

Promoter for high expression

AAAAGGTATTGACTTTCCCTACAGGGTGTGTAATAATTTATATACA
SPO1-15 [Lee, G and Pero, J, J. Mol. Biol., 152, 247 (1981)]
AAAAGTTGTTGACTTTATCTACAAGGTGTGGCATAATAATCTTAAC
SPO1-26 [Lee et al., Mol. Gen. Genet., 180, 57 (1980)]
GAAAAGTGTTGAAAATTGTCGAACAGGGTGATATAATAAAAGAGTA
φ29G36 [Marray, C. L. and Robinowitz, J. C., J. Biol. Chem., 257, 1053 (1982)]
GAAAAGGGTAGACAAACTATCGTTTAACATGTTATACTATAATAGAA
φ29G2 [Yoshikawa, H. et al., Proc. Natl. Acad. Sci., USA, 78, 1336 (1981)]
ATTAATGTTTGACAACTATTACAGAGTATGCTATAATGGTAGTATC
φ29A1 [Yoshikawa, H. et al., Proc. Natl. Acad. Sci., USA, 78, 1336 (1981)]
TAATATCGTTGACATTATCCATGTCCGTTGTTAAGATAAACATGAA
pur operon [Ebbole, D. J. and Zalkin, H., J. Biol. Chem., 262, 8274 (1987)]
Promoter for low expression
AATTTTATTTGACAAAAATGGGCTCGTGTTGTACAATAAATGTAGT
veg [Moran, C. P., Jr. et al., Mol. Gen. Genet., 186, 339 (1982)]
AAGTCTCCTTGAAATCAGAAGATATTTAGGATATATTTTTCTATGG

tms [Moran, C. P., Jr. et al., Mol. Gen. Genet., 186, 339 (1982)]
AAAAACGGTTGCATTTAAATCTTACATATCTAATACTTTCAAAGAC
penp [Himeno, T. et al., J. Bacteriol., 168, 1128 (1986)]
CAGTAATATTGACTTTTAAAAAAGGATTGATTCTAATGAAGAAAGC
cat [Horinovichi, S. and Weisblum, B., J. Bacteriol., 150, 815 (1982)]
AATTCCAAGTGTTAATATTCCTTAAAAAACATTTACTTCCATGGAAATGATGATAGATTA                ATTTTTAAGAAAAAGAACTGGTAATTCGCGAATTATGAAAAAGCGCTTTTTCTGCA
P1 [K. Nakahama et al., Gene, 36, 179 (1985)]

Such a promoter can be cut out from a DNA (plasmid) containing it, fractionated by, for example, agarose gel electrophoresis or polyacrylamide gel electrophoresis, and then recovered. Alternatively, a DNA having the base sequence of the above promoter can be synthesized by using a commercially available DNA synthesizer (e.g., an apparatus manufactured by Applied Biosystems Inc., U.S.A.) and following its protocol according to phosphoamide method.

Next, in order to obtain a large amount of the novel DNA of the present invention, a host microorganism having xanthine requirement is transformed with the above reaction mixture of ligation with T4DNA ligase to obtain a transformant wherein xanthine requirement of the host microorganism is complemented by expression of the promoter replaced. The preparation of a large amount of the DNA can be carried out from the transformant according to a method which itself has been known such as the method described in pages 86 to 93 of the above "Molecular Cloning".

The isolation of the DNA containing IMP dehydrogenase gene, wherein the promoter is replaced, from the plasmid thus obtained can be readily carried out by cleaving the plasmid with a restriction enzyme according to a conventional method, separating with agarose gel electrophoresis and then extracting and isolating with, for example, an electro-eluter.

Hereinafter, a method for the derivation of the novel microorganism by transformation of a recipient with the novel DNA thus obtained is illustrated.

As the recipient for this transformation, there can be used any microorganism, wherein a linear DNA provided outside of the cells can be incorporated therein and recombination is caused at a part on its chromosome having high homology to the base sequence of the DNA to change its inherent character, that is, any microbe having capability of transformation. The microorganism is not necessarily identical with the donor of IMP dehydrogenase gene in so far as the base sequence of its chromosomal DNA has high homology to that of IMP dehydrogenase gene contained in the linear DNA. For the preparation of a transformant having high capability of producing inosine and/or guanosine, bacteria belonging to the genus Bacillus such as Bacillus subtilis, Bacillus pumilus, Bacillus licheniformis are suitable from the viewpoints that it has been known that they accumulate two or more purine nucleotides and their related substances, for example, those selected from inosine, guanosine and xanthosine, they have capability of transformation, they have no pathogenicity, and they have a history that they have been used safely in fermentation industries.

Further, in many cases, a much improved result can be obtained, when a microorganism wherein one or more known characters advantageous for accumulation of purine nucleotides and their related substances, for example, purine analogue resistance such as 8-azaguanine resistance, nucleoside phosphorylase deficiency, 5′-guanylic acid reductase deficiency, folic acid antagonist resistance are provided to the above microorganism is used as the recipient.

Examples of the recipient of the genus Bacillus include as follows:
Bacillus subtilis BM 1032 (IFO 14559, FERM BP-1242)
Bacillus subtilis 1A3 (BGSC No. 1A3)
Bacillus subtilis NA-6011 (IFO 14189, FERM BP-291)
Bacillus subtilis NA-6012 (IFO 14190, FERM BP-292)
Bacillus subtilis NA-6128 (IFO 14373, FERM BP-617)
Bacillus subtilis NA-7821 (IFO 14368, FERM BP-618)
Bacillus subtilis ATCC 19221
Bacillus subtilis ATCC 14662
Bacillus pumilus (FERM P-2116)
Bacillus pumilus (FERM P-4832)
Bacillus pumilus (FERM P-4829)
Bacillus licheniformis IFO 12485

The novel DNA of the present invention is used for transformation of a microorganism belonging to the genus Bacillus and, in this case, it is possible to use it in an integrated form in a plasmid. However, in general, it is preferable to use it in a form of a linear DNA obtained by cleavage of a plasmid or cutting out of the DNA of the present invention.

The transformation of a microorganism of the genus Bacillus with the linear DNA fragment can be carried out according to a known method [C. Anagnostopoulos and J. Spizien, J. Bacteriol., 81, 741 (1961)].

In order to obtain the transformant of the present invention efficiently, for example, it is advantageous to render the recipient a xanthine requiring strain by previously deleting all or a part of the promoter of IMP dehydrogenase gene thereof. Thereby, the selection of the desired transformant from a large number of acceptor microorganism cells can be readily carried out by spreading and growing it on an agar plate containing no xanthine because a strain transformed with the DNA of the present invention is not a xanthine requiring strain. Such a mutant wherein the promoter and all or a part of the open reading frame of IMP dehydrogenase gene on the chromosome are deleted can be obtained by transforming a recipient with a DNA wherein the promoter and all or a part of the open reading frame of IMP dehydrogenase gene are deleted and selecting a xanthine requiring strain by replica plate method. The DNA for this purpose can be prepared from a plasmid containing IMP dehydrogenase gene having high homology to that of the recipient and an adjacent region, according to the same manner as the preparation of a DNA wherein the promoter is deleted provided that digestion with an exonuclease is further continued to delete not only the promoter but also all or a part of the structural gene, or by using a suitable restriction enzyme to prepare a DNA wherein the promoter and the open reading frame of the gene are deleted. In this case, preferably, conditions for digestion with an exonuclease are selected so that the promoter and the open reading frame of the gene are deleted as much as possible and the adjacent region is remained as much as possible. In the case of preparation of such a DNA with a restriction enzyme, preferably, an enzyme having a cleavage site which satisfies the same conditions as described above is selected. Further, in the case of the preparation of such a DNA, when a marker gene as described hereinafter is inserted into the deleted part, a xanthine requiring strain can be obtained by utilizing the marker, for example, drug resistance as a marker. Thereby, the selection is readily carried out and this is advantageous in practice. The insertion of a selection marker and collection of the transformant can be carried out, for example, according to the method described in European Patent Publication No. 273660 A. The xanthine requiring strain thus obtained is used as the recipient and the transformation is carried out with the DNA of the present invention. Then, a cell suspension is spread on an agar plate containing no xanthine and the novel microorganism of the present invention can be obtained from a colony grown on the plate.

The transformant thus obtained is a novel microorganism. This can be shown as follows. That is, each of the transformant and the chromosomal DNA of the recipient used for transformation is cleaved with a suitable restriction enzyme, subjected to agarose gel electrophoresis, denatured with an alkali and transferred to a nitrocellulose filter. On the other hand, a fragment containing IMP dehydrogenase gene and a DNA fragment containing the promoter used are labeled with a radioisotope, respectively. By using them as probes, Southern hybridization is carried out and the size of the resulting fragment hybridized with each probe is analyzed to confirm that the promoters are exchanged. Further, the exchange of the promoters can be also confirmed by examining the DNA sequence of the promoter region in IMP dehydrogenase gene of the chromosomal DNA of the transformant. When double crossing-over type recombination is caused upon transformation with the DNA of the present invention, there is difference in the strength of expression of IMP dehydrogenase gene. Further, when single crossing-over type recombination is caused by the DNA of the present invention, the character of the marker used is expressed in addition to the above character. However, other microbiological characters are the same as those of the recipient.

Typical examples of the transformant of the present invention include

Bacillus subtilis NA6242 (IFO 14867, FERM BP-2381)

Bacillus subtilis NA6243 (IFO 14868, FERM BP-2382)

obtained in the Examples hereinafter. The IFO number is the accession number in Institute for Fermentation, Osaka and FERM BP-number is the accession number in Fermentation Research Institute, Agency of Industrial Science and Technology (FRI) under Budapest treaty. Each transformant is deposited on April 3, 1989 with IFO and on April 12, 1989 with FRI.

Of course, by selecting the promoter and the recipient appropriately, various transformants can be readily prepared according to the method as described herein.

In order to produce inosine and/or guanosine by using the transformant obtained by the present invention, there can be used a conventional fermentation process for the production of inosine and/or guanosine.

That is, culture media containing carbon sources, nitrogen sources, metal ions and, if necessary, other nutrients such as amino acids, nucleic acids, vitamins and the like can be used. As the carbon sources, glucose, sucrose, maltose, starch, saccharified starch, molasses and the like can be used. They can be used alone or in combination thereof. As the nitrogen sources, in addition to organic nitrogen sources such as peptone, soybean meal, dried yeast, urea and the like, inorganic nitrogen sources such as ammonium salts of sulfuric acid, nitric acid, hydrochloric acid, carbonic acid and the like, gaseous ammonia, aqueous ammonia and the like can be also used alone or in combination thereof. As other nutrients, various inorganic salts, amino acids, vitamins and the like which are necessary for the growth of the microorganism are appropriately selected and used alone or in combination thereof. As adenine source, there can be used adenine, adenosine and adenylic acid as well as microbial cells containing them and their extracts.

Further, if necessary, an antifoaming agent such as silicone oil, polyalkylene glycol ether or the like, a surfactant and the like can be added to the culture medium.

Usually, the cultivation is carried out under aerobic conditions such as aeration submerged culture and the like. Preferably, pH of the culture medium is within the range of 4 to 9. When pH is varied during cultivation, sulfuric acid, calcium carbonate, sodium hydroxide, gaseous ammonia, aqueous ammonia and the like can be added to adjust pH to the above preferred range. Usually, the cultivation temperature is appropriately selected from the range of 20 to 45° C so that the temperature is suitable for the growth of the particular microorganism to be used and the accumulation of inosine and/or guanosine. The cultivation is continued until the accumulation of inosine and/or guanosine substantially becomes maximum. Usually, the desired result can be obtained by cultivation for 24 to 144 hours.

In order to isolate and obtain inosine and/or guanosine from a culture, there can be employed known purification means, for example, precipitation and isolation and purification methods such as chromatography using an ion exchange resin, activated charcoal and the like.

According to the present invention, an accumulation ratio of either inosine or guanosine which is a starting material for the production of an important flavor component, 5′-inosinic acid or 5′-guanylic acid, can be increased and an industrially advantageous production thereof can be realized with high yield.

That is, a novel microorganism having a high capability of the production of guanosine can be obtained by using the microorganism of the genus Bacillus being capable of producing inosine or guanosine as the recipient and transforming it with the DNA of the present invention. Since IMP dehydrogenase gene having a promoter of high expression is integrated on the chromosome of the novel microorganism, a very stable production can be carried out, repeatedly. Further, according to the present invention, the novel microorganism having high capability of the production of inosine can be also obtained by using a microorganism of the genus Bacillus being capable of producing inosine and guanosine, or inosine and xanthosine, or xanthosine and/or guanosine as the recipient and transforming it with the other DNA of the present invention. Since the promoter of the IMP dehydrogenase gene on the chromosome of the latter novel microorganism is modified, a very stable production of inosine can be carried out, repeatedly.

The following Reference Example and Examples further illustrate the present invention in detail but are not to be construed to limit the scope thereof.

In the Reference Examples and Examples, all the restriction enzymes (all manufactured by Nippon Gene Co., Ltd., Japan) and modification enzymes were used in the amount of 5 U per 1 μg of DNA. The reaction conditions were followed to those specified by the manufacturer unless otherwise stated.

Reference Example 1

Isolation of the plasmid pEX117 containing IMP dehydrogenase gene of Bacillus subtilis

The recombinant plasmid pEX117 containing an IMP dehydrogenase gene region derived from Bacillus subtilis NA-6012 (IFO 14190, FERM BP-292) was extracted from a transformant containing the plasmid, Escherichia coli TEX 117 (Japanese Patent Laid Open Publication No. 60-156388), according to the method described in pages 86 to 93 of the above "Molecular Cloning". Namely, Escherichia coli TEX 117 was inoculated to LB medium (Bacto tryptone: 10 g/liter, yeast extract: 5 g/liter, sodium chloride: 5 g/liter) and 140 μg/ml of chloramphenicol was added thereto to amplify the plasmid. After cultivation overnight, the cells are collected and washed. Lysozyme was added to the washed cells and further 0.2 N sodium hydroxide solution containing 1% of sodium lauryl sulfate to cause lysis of the cells. After addition of 5 M potassium acetate, a supernatant containing the plasmid was obtained by centrifugation. To the supernatant was added 0.6 volume of isopropanol to precipitate the plasmid DNA and, after washing with ethanol, it was dissolved in TE buffer (10 mM Tris-HCl buffer-1 mM EDTA, pH 8.0). To the solution was added cesium chloride so that the specific gravity became 1.60 and further added ethidium bromide so that the final concentration became 600 μg/ml. The mixture was centrifuged at 50,000 rpm at 20° C by an ultracentrifuge (rotor: V₆₅ Ti) and a band of the plasmid detected by ultraviolet rays was taken out. After removing ethidium bromide with n-butanol, it was dialized against TE buffer to obtain about 200 μg of the recombinant plasmid pEX117 from 300 ml of the culture solution.

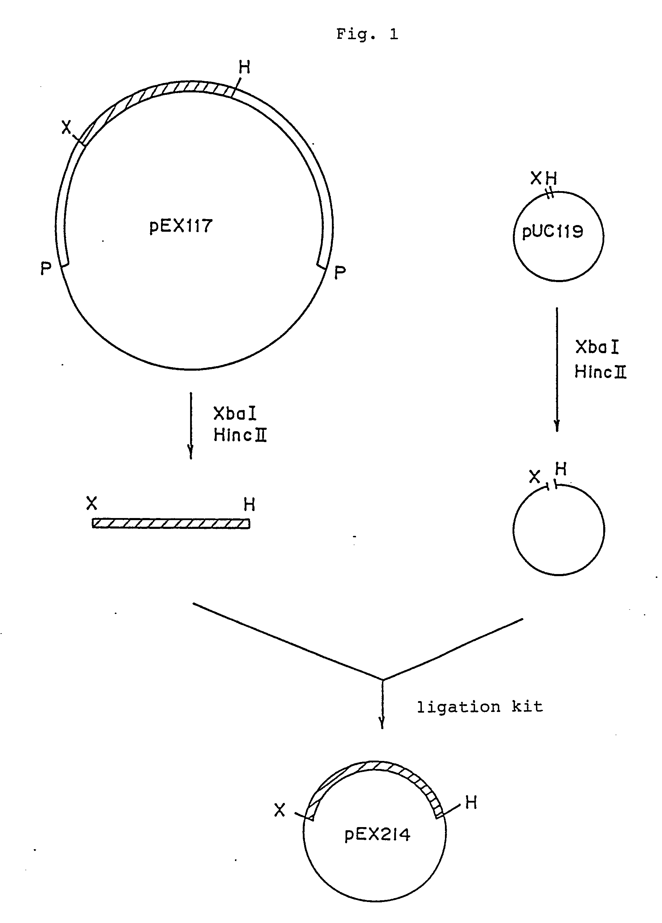

pEX117 was cleaved with various restriction enzymes and the restriction map as shown in Fig. 1 was obtained from agarose gel electrophoresis pattern based on the molecular weight of HindIII digested lambda phage DNA.

pEX117 is a recombinant plasmid wherein a DNA fragment of about 6.4 k base pair (bp) is inserted into PstI site of pBR322.

Example 1

Preparation of the novel DNA wherein the promoter a part of IMP dehydrogenase gene is replaced with SPO1

1-i) Subcloning of IMP dehydrogenase gene

pEX117 (3 μg) was double-digested with XbaI and HincII. On the other hand, pUC119 (1 μg) was double-digested with XbaI and HincII. They were mixed and 1/2 volume of 7.5 M ammonium acetate was added to the mixture. Further, ethanol was added so that the final concentration became 70% to precipitate DNA's. After centrifugation, the DNA's were dissolved in TE buffer (5 μl) and ligated to each other with a ligation kit (manufactured by Takara Shuzo Co., Ltd., Japan). A part of this reaction mixture (5 μl) was used to transform Escherichia coli X 895 (IFO 14308, FERM P-7411, xanthine requiring strain) and a cell suspension was spread on M-9 CATA medium (Na$_2$HPO$_4$: 6 g/liter, KH$_2$PO$_4$: 3 g/liter, NaCl: 0.5 g/liter, NH$_4$Cl: 1 g/liter, MgSO$_4$: 1 mM, CaCl$_2$: 1 mM, glucose: 2 g/liter, Casamino acid: 2 g/liter, tryptophane: 50 mg/ml, adenine: 50 μg/ml, agar: 15 g/liter, pH 7.2) containing ampicillin (25 μg/ml). After incubation at 37°C for one day, a plasmid was extracted from the transformant grown on the plate according to a conventional method and named as pEX214. The transformation was carried out according to the method of S. N. Cohen et al. [Proc. Natl. Acad. Sci. USA, 69, 2110 (1972)]. Then, according to the same manner as described in Reference Example 1, a large amount of the DNA was prepared and 1300 μg of the DNA was obtained from 1 liter of the culture solution.

The construction of pEX214 is illustrated in Fig. 1.

1-ii) Determination of the DNA sequence of IMP dehydrogenase gene contained in pEX214

According to the method of N. Poncz et al. [Proc. Natl. Acad. Sci. USA, 79, 4298 (1982)], DNA fragments having deletion were prepared with respect to the XbaI-HincII fragment of pEX214 and, according to Sanger method, the base sequences thereof were determined. For carrying out Sanger method, SEQUENASE (manufactured by United States Biochemical Corp., U.S.A.) was used by following the protocol of the manufacturer.

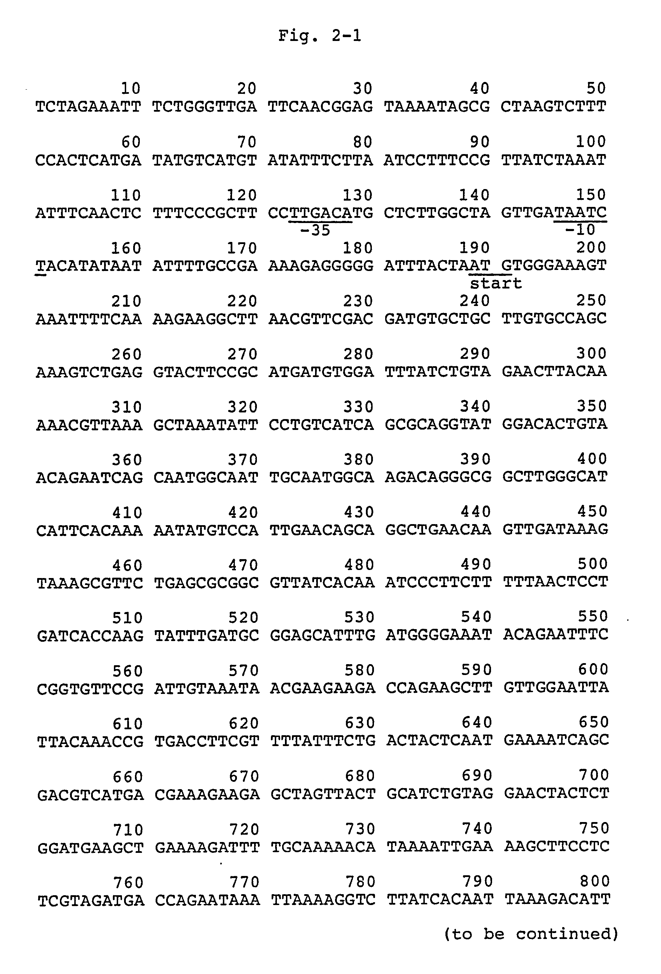

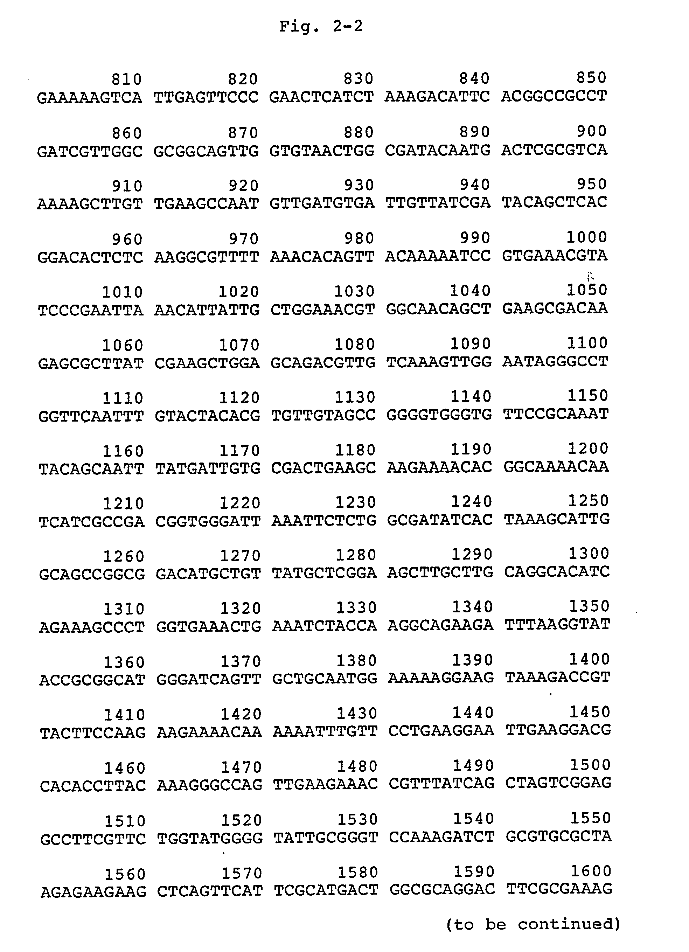

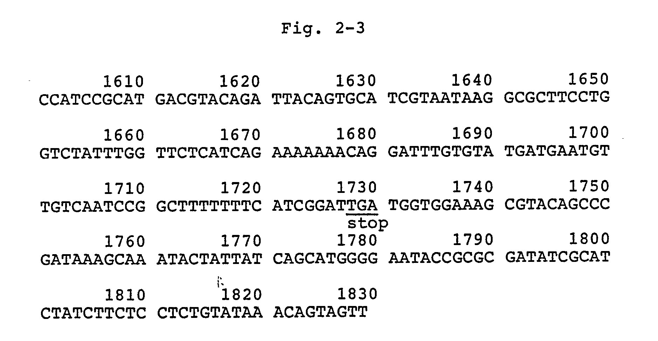

The result of the base sequence determination is shown in Fig. 2.

1-iii) Deletion of the promoter of IMP dehydrogenase gene

After cleaving pEX214 (10 μg) with XbaI, DNA was recovered by ethanol precipitation and dissolved in TE buffer (10 μl). Then, the buffer, water and BAL31 (0.2 U) were added to bring the reaction mixture to 50 μl and the mixture was incubated at 30°C. Seven μl portions of the reaction mixture were taken out every one minute and TE buffer saturated phenol (7 μl) was added to each portion. From the solution, DNA was recovered by ethanol precipitation and dissolved in TE buffer (5 μl). Then, the bonding end was converted into a blunt end with T4DNA polymerase. PstI was added to 7 DNA solutions thus obtained and, after digestion, each of them was subjected to agarose gel electrophoresis. Agar containing the fragment corresponding to a DNA having the degree of electrophoresis of about 1.7 kbp (1.6 to 1.75 kbp) was cut out and the DNA fragment was recovered from the agar with an electro-eluter (manufactured by IBI Corp., U.S.A.). Such a fragment was obtained from a sample of one minute reaction with BAL31.

On the other hand, pUC119 (1 μg) was double-digested with SmaI and PstI and, according to the same manner, a DNA fragment corresponding to about 3.2 kbp was recovered.

Both DNA's were mixed and ligated with the ligation kit and used for transformation of Escherichia coli JM 109. The cell suspension was spread on a LB agar plate containing ampicillin and incubated at 37°C for one day, and 12 transformants grown on the plate were collected. According to the same manner as described above 1-ii), the base sequence of the inserted part on the plasmid contained in each transformant was examined to select the plasmid pEX214Δ3 wherein -35 region was deleted.

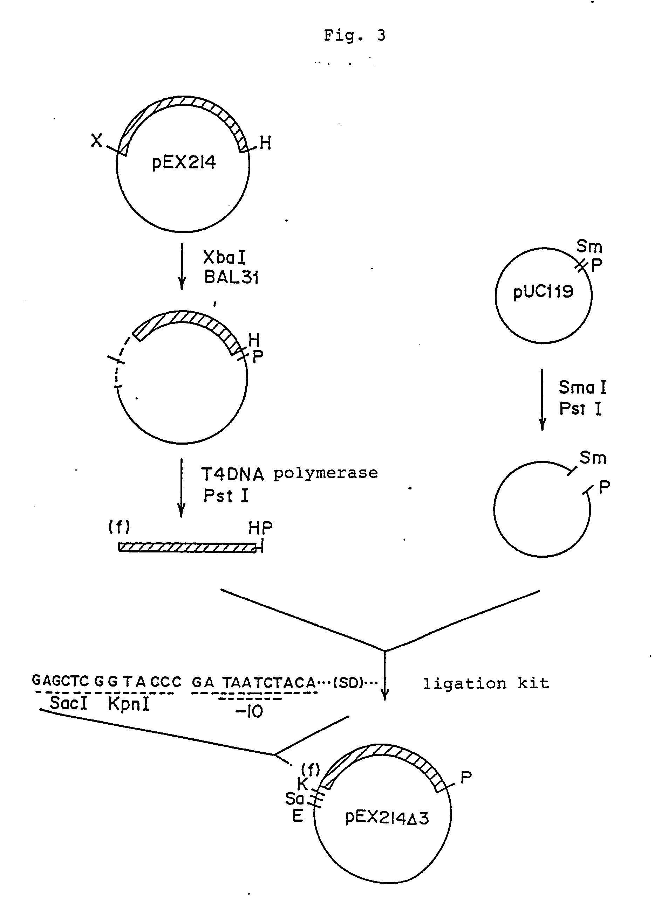

The construction of pEX214Δ3 and the determination of its base sequence are illustrated in Fig. 3.

1-iv) Insertion of a 5'-adjacent region of IMP dehydrogenase gene into pEX214Δ3

pEX117 (5 μg) was double-digested with SmaI and XbaI. pEX214 (20 μg) was cleaved with SspI. After fractionation of each of them by agarose gel electrophoresis, the fragment of 1.45 kbp (about 1 μg) was recovered from the former and the fragment of 1.2 kbp (about 4 μg) was recovered from the latter. The SspI fragment of 1.2 kbp was further cleaved with XbaI and fractionated according to the same manner to recover the fragment of 0.1 kbp (about 0.2 μg). On the other hand, after cleaving pEX214Δ3 (1 μg) with EcoRI, the cohesive ends were converted into blunt ends with T4DNA polymerase according to the description of page 395 of "Molecular Cloning". To this were added the above recovered fractions of 1.45 kbp (1 μg) and 0.1 kbp (0.2 μg) and these three fragments were ligated with the ligation kit. By using a part of the reaction mixture (5 μl), Escherichia coli X895 strain was transformed. A plasmid was extracted from the transformant grown on a LB agar plate containing ampicillin and named as pEX241.

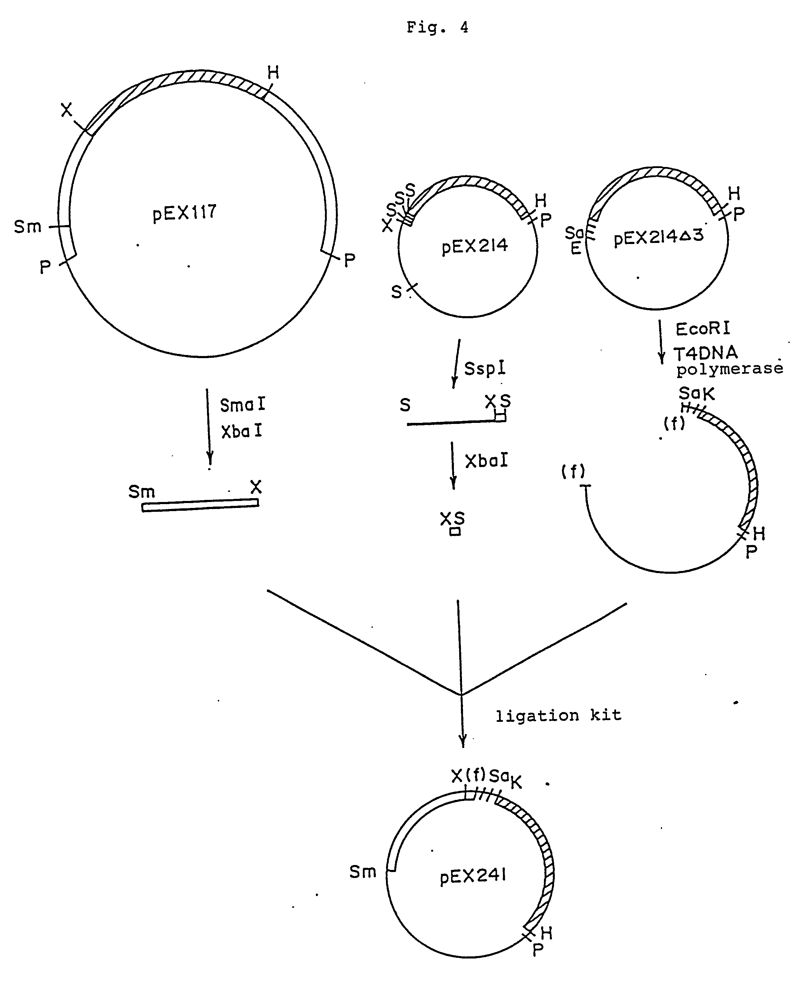

The construction of pEX241 is illustrated in Fig. 4.

1-v) Ligation of SPO-1 promoter to pEX241

The plasmid pSPO1-26 (10 μg) containing SPO1-26 promoter was cleaved with EcoRI and fractionated by agarose gel electrophoresis to recover a DNA fragment of about 0.1 kbp. This was dissolved in TE buffer (5 μl). On the other hand, pEX241 (1 μg) was cleaved with KpnI, mixed with the above fragment of 0.1 kbp. Then, the cohesive ends were converted into blunt ends by T4DNA polymerase. They were ligated with the ligation kit and used for transformation of Escherichia coli X895. The transformation reaction mixture was spread on a M-9 CATA agar plate containing ampicillin and the transformant, Escherichia coli TF242 (IFO 14864, FERM BP-2378), was obtained from a colony grown on the plate. According to the same manner as described in Reference Example 1, a plasmid (about 1,200 μg) was obtained from the culture solution and named as pEX242. pEX242 (50 μg) was double-digested with PvuI and PstI. After fractionation by agarose gel electrophoresis, a fragment of 2.9 kbp was recovered (yield: about 7 μg).

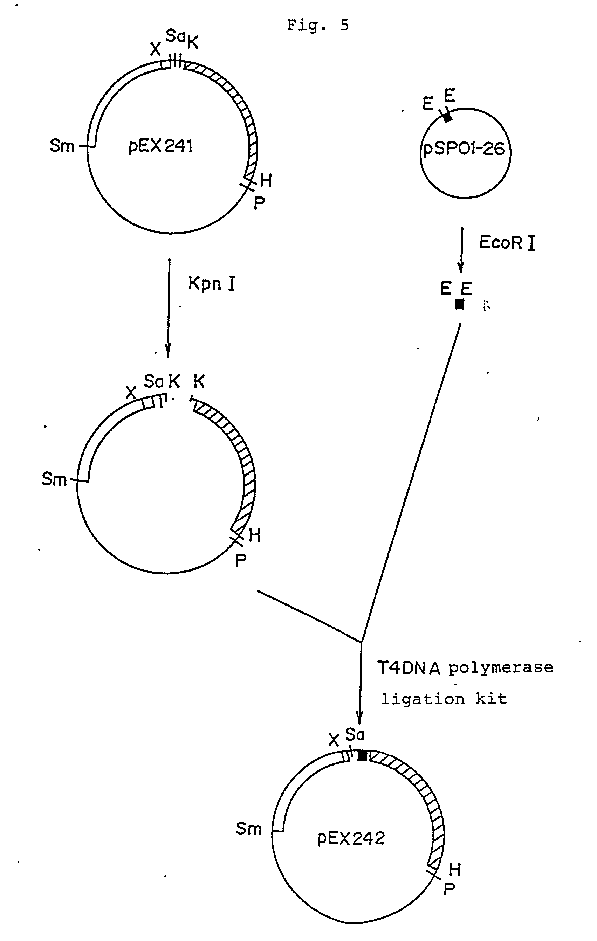

The construction of pEX242 is illustrated in Fig. 5.

Example 2

2-i) Preparation of a plasmid containing DNA wherein almost part of IMP dehydrogenase gene is deleted

pEX117 (5 μg) was double-digested with XbaI and MluI. After fractionation by agarose gel electrophoresis, a fragments of 7.0 kbp and 3.8 kbp were recovered. The fragment of 3.8 kbp was further digested with NruI to recover a NruI-MluI fraction. On the other hand, according to the same manner as described in Example 1, a XbaI-SspI fragment (0.1 kbp) was recovered from pEX214.

Further, pC194 (5 μg) was digested with ClaI to recover a ClaI fragment of 1.7 kbp. This was further double-digested with MspI and MboI to recover a fragment of 1.05 kps which contained chloramphenicol acetyltransferase gene. The cohesive ends of the fragment were converted into blunt ends with T4DNA polymerase.

Then, 4 fragments obtained by these operations, namely,

XbaI-MluI fragment of 7.0 kbp
NruI-MluI fragment of 2.2 kbp
XbaI-SspI fragment of 0.1 kbp
MspI-MboI fragment of 1.05 kbp (both ends were converted into blunt ends)

were taken out in the amounts of 0.5 μg, 0.5 μg, 0.1 μg and 0.2 μg, respectively, mixed and ligated with a ligation kit. A part of this reaction mixture was used for transformation of Escherichia coli C600 and a cell suspension was spread on a LB agar plate containing chloramphenicol (10 μg/ml) and tetracycline (12.5 μg/ml). After incubation at 37°C for one day, a plasmid was extracted from a strain grown on the plate according to a conventional method and named as pEX210.

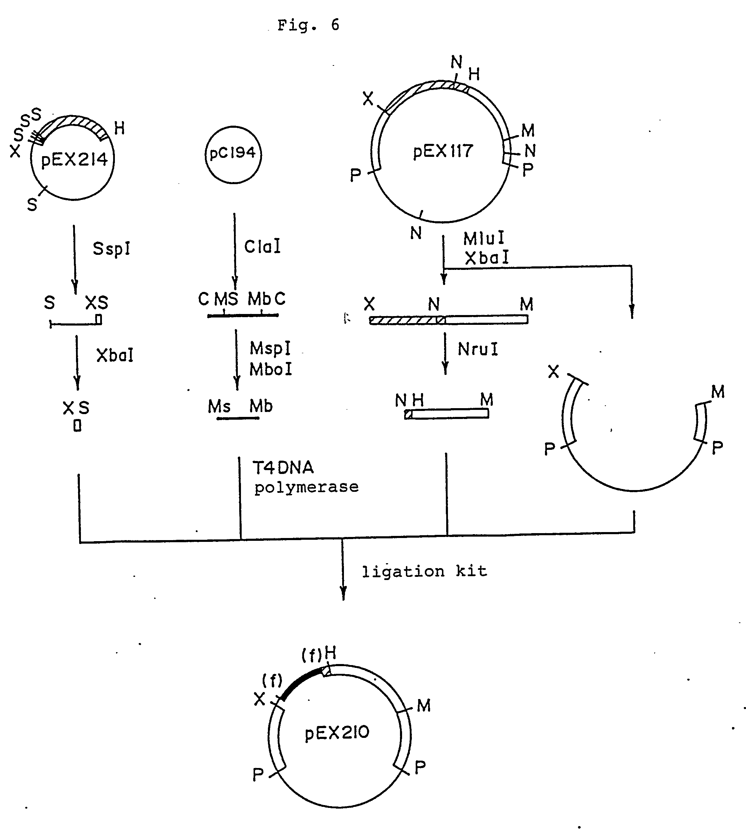

The construction of pEX210 is illustrated in Fig. 6.

2-ii) Derivation of a transformant wherein almost part of IMP dehydrogenase on the chromosome was deleted

pEX210(50 μg) was cleaved with PstI and, according to the same manner as described in Example 1-v), a fragment of 7.0 kbp was isolated and recovered. This fragment (1 μg) was added to a competent cell suspension (1 ml) of Bacillus subtilis NA6128 (IFO 14373, FERM BP-617) prepared according to the method of C. Anagnostopoulos and J. Spizizen [J. Bacteriol., 81, 741 (1961). After cultivation with shaking at 37°C for one hour, the culture was spread on LB agar plate containing chloramphenicol (10 μg/ml) and incubated at 37°C for one day. The resulting transformant was collected and named as Bacillus subtilis NA6210 (IFO 14866, FERM BP-2380). The microbial strain was inoculated on a M-9 CATA agar plate and a M-9 CATA agar plate containing xanthine (50 μg/ml) and growth on both media was observed. As the result, it was found that this strain did not grow on the former plate, whereas it grew on the latter plate. In view of this, it was clear that NA6210 strain became a xanthine requiring strain.

2-iii) Derivation of a transformant carrying IMP dehydrogenase gene wherein the promoter of the gene is replaced with SPO1-26 promoter

Competent cells of Bacillus subtilis NA6210 strain was prepared and to a suspension thereof (1 ml) was added PvuI-PstI fragment of pEX242 prepared in Example 1-v) (2.9 kbp, 1 μg). The mixture was shaken at 37°C for one hour. Then, the cell suspension was spread on M-9 CATA agar plate and incubated at 37°C for one day. A colony grown on the plate was collected to obtain Bacillus subtilis NA6242 strain (IFO 12485, FERM BP-2381). The growth of this strain on a LB agar plate and a LB agar plate containing chloramphenicol (10 μg/ml) was observed. As the result, it was found that this strain grew on the former plate, whereas it did not grow on the latter plate. Thus, this strain was sensitive to chloramphenicol, although NA6210 strain was resistant to chloramphenicol.

2-iv) Comparison of IMP dehydrogenase activities of Bacillus subtilis NA6128 and NA6242

When IMP dehydrogenase activity of Bacillus subtilis NA6242 was measured by the above method described by Magasanik, it was 13 nmol of XMP formed/min. mg protein. On the other hand, when IMP dehydrogenase activity of Bacillus subtilis NA6128 was measured according to the same manner, it was 1.5 nmol of XMP formed/min. mg protein. Thus, the enzymatic activity increased about 8.7 times due to the replacement of the promoter.

Example 3

Production of guanosine by Bacillus subtilis NA6242

One loopful of Bacillus subtilis NA6242 was inoculated in a 200 ml volume Erlenmeyer flask containing a seed culture medium (20 ml) as shown in Table 1 and cultivated with shaking at 37°C for 18 hours. One ml thereof was inoculated in a 200 ml creased flask containing a main fermentation medium (20 ml) as shown in Table 1 and cultivated on a rotary shaker at 37°C for 84 hours. After completion of the cultivation, the amount of guanosine accumulated was determined by high performance liquid chromatography. As the result, it was found that 24.0 mg/ml of guanosine was accumulated. When Bacillus subtilis NA6128 was cultivated according to the same manner, only 8.0 mg/ml of guanosine was accumulated.

Table 1

| Components of medium | Concentration (g/liter) | |
|---|---|---|
| | Seed culture medium | Fermentation medium |
| Glucose | 30 | 120 |
| Sodium glutamate | 10 | 10 |
| Ammonium sulfate | - | 20 |
| Urea | 3 | 10 |
| Corn steep liquor | 20 | 20 |
| Magnesium sulfate | 2 | 2 |
| Adenine | 0.2 | 0.075 |
| Dipotassium hydrogenphosphate | 21 | - |
| Potassium chloride | - | 0.5 |
| Calcium chloride | 2 | 5 |
| Manganese sulfate | - | 0.0025 |
| Calcium carbonate | - | 30 |
| pH | 7.0 | 6.8 |

Example 4

Insertion of P1 promoter to pEX241

A DNA fragment containing P1 promoter was cut out from the plasmid pBTM128 containing P1 promoter prepared according to the method as described in Japanese Patent Laid Open Publication No. 60-137291 and ligated to pEX241 according to the same manner as described in Example 1-v). Namely, pBTM128 (10 $\mu$g) was double-digested with EcoRI and PstI and, after isolation by agarose gel electrophoresis, a DNA fragment of about 0.1 kbp was recovered. It was dissolved in TE buffer (5 $\mu$l). On the other hand, pEX241 (1 $\mu$g) was cleaved with KpnI and mixed with the above 0.1 kbp fragment. The cohesive ends were converted into blunt ends with T4DNA polymerase. Then, they were ligated with a ligation kit and used for transformation of Escherichia coli X895. A cell suspension was spread on M-9 CATA agar plate and incubated at 37°C overnight. From a colony formed on the plate, the transformant, Escherichia coli TF243 (IFO 14865, FERM BP-2379) was obtained. According to the same manner as described in Reference Example 1, about 1,200 $\mu$g of the plasmid was extracted from 1 liter of the culture solution and named as pEX243. pEX243 was double-digested with PvuI and PstI and, after fractionation by agarose gel electrophoresis, a fragment of 2.9 kbp was recovered to obtain about 7 $\mu$g of DNA.

Example 5

Derivation of transformant carrying IMP dehydrogenase gene wherein the promoter of the gene was replaced with p1 promoter

To the competent cells of Bacillus subtilis NA-6210 prepared in Example 2 was added PvuI-PstI fragment of pEX243 (2.9 kbp, 7 $\mu$g) prepared in Example 4 and transformation was carried out according to the same manner as described in Example 2-iii). A cell suspension was spread on a M-9 CATA agar plate and, from a colony formed, the transformant, Bacillus subtilis NA6243 (IFO 14868, FERM BP-2382) was obtained. This strain was examined by the method as described in Example 2-iii) and it was found that the strain was sensitive to chloramphenicol.

According to the same manner as described in Example 2-iv), when IMP dehydrogenase activity of Bacillus subtilis NA6243 was measured, it was 0.5 nmol of XMP formed/min. mg protein and, therefore, the enzymatic activity was reduced to about 1/3 due to the replacement of the promoter.


Example 6


Production of inosine by Bacillus subtilis NA6243

One loopful of Bacillus subtilis NA6243 was inoculated in a 200 ml volume Erlenmeyer flask containing the seed culture medium (20 ml) as shown in Table 1 and cultivated with shaking at 37°C for 18 hours. One ml of the culture was inoculated in a 200 ml volume creased flask containing the main fermentation medium (20 ml) as shown in Table 1 and cultivated on a rotary shaker at 37°C for 84 hours. When the amounts of inosine and guanosine accumulated were determined according to the same manner as described in Example 3 at the end of fermentation, accumulation of 27.0 mg/ml of inosines and 3.0 mg/ml of guanosine was observed. When Bacillus subtilis NA6128 was cultivated according to the same manner, only 22.0 mg/ml of inosine and 8.0 mg/ml of guanosine were accumulated.


## Claims

1. A DNA containing IMP dehydrogenase gene of the chromosome of a microorganism belonging to the genus Bacillus wherein a promoter of the IMP dehydrogenase gene is replaced with a different expressible promoter.

2. A DNA according to claim 1, wherein the different promoter is a member selected from the group consisting of those derived from plasmids of chromosomes of bacteria belonging to the genus Bacillus, plasmids derived from phage of bacteria belonging to the genus Bacillus, and plasmids being capable of autonomous growth in bacteria belonging to the genus Bacillus.

3. A DNA according to claim 1, wherein the promoter of the IMP dehydrogenase gene is replaced with a promoter which can express stronger than IMP dehydrogenase gene on the chromosome of a microorganism belonging to the genus Bacillus.

4. A DNA according to claim 1, wherein the promoter of the IMP dehydrogenase gene is replaced with a promoter which can express weaker than IMP dehydrogenase gene on the chromosome of a microorganism belonging to the genus Bacillus.

5. A vector integrated with a DNA containing IMP dehydrogenase gene of the chromosome of a microbe belonging to the genus Bacillus wherein a promoter of the IMP dehydrogenase gene is replaced with a different expressible promoter.

6. A vector according to claim 5, wherein the different promoter is a member selected from the group consisting of those derived from plasmids of chromosomes of bacteria belonging to the genus Bacillus, plasmids derived from phage of bacteria belonging to the genus Bacillus, and plasmids being capable of autonomous growth in bacteria belonging to the genus Bacillus.

7. A vector according to claim 5, wherein the promoter of the IMP dehydrogenase gene is replaced with a promoter which can express stronger than IMP dehydrogenase gene on the chromosome of a microorganism belonging to the genus Bacillus.

8. A vector according to claim 5, wherein the promoter of the IMP dehydrogenase gene is replaced with a promoter which can express weaker than IMP dehydrogenase gene on the chromosome of a microorganism belonging to the genus Bacillus.

9. A microorganism belonging to the genus Bacillus fitted with a gene, wherein a promoter of a gene on the chromosome of a microorganism belonging to the genus Bacillus is replaced with a different expressible promoter, on the chromosome thereof.

10. A microorganism belonging to the genus Bacillus being capable of producing inosine and/or guanosine according to claim 9, which is transformed with a DNA containing IMP dehydrogenase gene of the chromosome of a microbe belonging to the genus Bacillus wherein a promoter of the IMP dehydrogenase gene is replaced with a different expressible promoter or a vector integrated with the DNA.

11. A microorganism according to claim 10, wherein the different promoter is a member selected from the group consisting of those derived from plasmids of chromosomes of bacteria belonging to the genus Bacillus, plasmids derived from phage of bacteria belonging to the genus Bacillus, and plasmids being capable of autonomous growth in bacteria belonging to the genus Bacillus.

12. A microorganism according to claim 10, wherein the promoter of the IMP dehydrogenase gene is

EP 0 393 969 A2

replaced with a promoter which can express stronger than IMP dehydrogenase gene on the chromosome of a microorganism belonging to the genus Bacillus.

13. A microorganism according to claim 10, wherein the promoter of the IMP dehydrogenase gene is replaced with a promoter which can express weaker than IMP dehydrogenase gene on the chromosome of a microorganism belonging to the genus Bacillus.

14 A process for producing inosine and/or guanosine which comprises cultivating the microorganism of the genus Bacillus being capable of producing inosine and/or guanosine which is transformed with a DNA containing IMP dehydrogenase gene of the chromosome of a microbe belonging to the genus Bacillus wherein a promoter of the IMP dehydrogenase gene is replaced with a different expressible promoter or a vector integrated with the DNA in a culture medium to produce and accumulate inosine and/or guanosine and collecting the resultant.

15. A process according to claim 14, wherein the different promoter is a member selected from the group consisting of those derived from plasmids of chromosomes of bacteria belonging to the genus Bacillus, plasmids derived from phage of bacteria belonging to the genus Bacillus, and plasmids being capable of autonomous growth in bacteria belonging to the genus Bacillus.

16. A process according to claim 14, wherein the promoter of the IMP dehydrogenase gene is replaced with a promoter which can express stronger than IMP dehydrogenase gene on the chromosome of a microorganism belonging to the genus Bacillus.

17. A process according to claim 14, wherein the promoter of the IMP dehydrogenase gene is replaced with a promoter which can express weaker than IMP dehydrogenase gene on the chromosome of a microorganism belonging to the genus Bacillus.

14

Fig. 1

Fig. 2-1

```
        10         20         30         40         50
TCTAGAAATT TCTGGGTTGA TTCAACGGAG TAAAATAGCG CTAAGTCTTT

        60         70         80         90        100
CCACTCATGA TATGTCATGT ATATTTCTTA ATCCTTTCCG TTATCTAAAT

       110        120        130        140        150
ATTTCAACTC TTTCCCGCTT CCTTGACATG CTCTTGGCTA GTTGATAATC
                             -35                     -10
       160        170        180        190        200
TACATATAAT ATTTTGCCGA AAAGAGGGGG ATTTACTAAT GTGGGAAAGT
                                            start
       210        220        230        240        250
AAATTTTCAA AAGAAGGCTT AACGTTCGAC GATGTGCTGC TTGTGCCAGC

       260        270        280        290        300
AAAGTCTGAG GTACTTCCGC ATGATGTGGA TTTATCTGTA GAACTTACAA

       310        320        330        340        350
AAACGTTAAA GCTAAATATT CCTGTCATCA GCGCAGGTAT GGACACTGTA

       360        370        380        390        400
ACAGAATCAG CAATGGCAAT TGCAATGGCA AGACAGGGCG GCTTGGGCAT

       410        420        430        440        450
CATTCACAAA AATATGTCCA TTGAACAGCA GGCTGAACAA GTTGATAAAG

       460        470        480        490        500
TAAAGCGTTC TGAGCGCGGC GTTATCACAA ATCCCTTCTT TTTAACTCCT

       510        520        530        540        550
GATCACCAAG TATTTGATGC GGAGCATTTG ATGGGGAAAT ACAGAATTTC

       560        570        580        590        600
CGGTGTTCCG ATTGTAAATA ACGAAGAAGA CCAGAAGCTT GTTGGAATTA

       610        620        630        640        650
TTACAAACCG TGACCTTCGT TTTATTTCTG ACTACTCAAT GAAAATCAGC

       660        670        680        690        700
GACGTCATGA CGAAAGAAGA GCTAGTTACT GCATCTGTAG GAACTACTCT

       710        720        730        740        750
GGATGAAGCT GAAAAGATTT TGCAAAAACA TAAAATTGAA AAGCTTCCTC

       760        770        780        790        800
TCGTAGATGA CCAGAATAAA TTAAAAGGTC TTATCACAAT TAAAGACATT
```

(to be continued)

Fig. 2-2

```
       810        820        830        840        850
GAAAAAGTCA TTGAGTTCCC GAACTCATCT AAAGACATTC ACGGCCGCCT

       860        870        880        890        900
GATCGTTGGC GCGGCAGTTG GTGTAACTGG CGATACAATG ACTCGCGTCA

       910        920        930        940        950
AAAAGCTTGT TGAAGCCAAT GTTGATGTGA TTGTTATCGA TACAGCTCAC

       960        970        980        990       1000
GGACACTCTC AAGGCGTTTT AAACACAGTT ACAAAAATCC GTGAAACGTA

      1010       1020       1030       1040       1050
TCCCGAATTA AACATTATTG CTGGAAACGT GGCAACAGCT GAAGCGACAA

      1060       1070       1080       1090       1100
GAGCGCTTAT CGAAGCTGGA GCAGACGTTG TCAAAGTTGG AATAGGGCCT

      1110       1120       1130       1140       1150
GGTTCAATTT GTACTACACG TGTTGTAGCC GGGGTGGGTG TTCCGCAAAT

      1160       1170       1180       1190       1200
TACAGCAATT TATGATTGTG CGACTGAAGC AAGAAAACAC GGCAAAACAA

      1210       1220       1230       1240       1250
TCATCGCCGA CGGTGGGATT AAATTCTCTG GCGATATCAC TAAAGCATTG

      1260       1270       1280       1290       1300
GCAGCCGGCG GACATGCTGT TATGCTCGGA AGCTTGCTTG CAGGCACATC

      1310       1320       1330       1340       1350
AGAAAGCCCT GGTGAAACTG AAATCTACCA AGGCAGAAGA TTTAAGGTAT

      1360       1370       1380       1390       1400
ACCGCGGCAT GGGATCAGTT GCTGCAATGG AAAAAGGAAG TAAAGACCGT

      1410       1420       1430       1440       1450
TACTTCCAAG AAGAAAACAA AAAATTTGTT CCTGAAGGAA TTGAAGGACG

      1460       1470       1480       1490       1500
CACACCTTAC AAAGGGCCAG TTGAAGAAAC CGTTTATCAG CTAGTCGGAG

      1510       1520       1530       1540       1550
GCCTTCGTTC TGGTATGGGG TATTGCGGGT CCAAAGATCT GCGTGCGCTA

      1560       1570       1580       1590       1600
AGAGAAGAAG CTCAGTTCAT TCGCATGACT GGCGCAGGAC TTCGCGAAAG
```

(to be continued)

Fig. 2-3

```
      1610          1620          1630          1640          1650
CCATCCGCAT GACGTACAGA TTACAGTGCA TCGTAATAAG GCGCTTCCTG


      1660          1670          1680          1690          1700
GTCTATTTGG TTCTCATCAG AAAAAAACAG GATTTGTGTA TGATGAATGT


      1710          1720          1730          1740          1750
TGTCAATCCG GCTTTTTTTC ATCGGATTGA TGGTGGAAAG CGTACAGCCC
                               stop
      1760          1770          1780          1790          1800
GATAAAGCAA ATACTATTAT CAGCATGGGG AATACCGCGC GATATCGCAT


      1810          1820          1830
CTATCTTCTC CTCTGTATAA ACAGTAGTT
```

Fig. 3

EP 0 393 969 A2

Fig. 4

Fig. 5

EP 0 393 969 A2

Fig. 6